(19) Europäisches Patentamt / European Patent Office / Office européen des brevets

(11) **EP 4 129 968 B1**

(12) **EUROPEAN PATENT SPECIFICATION**

(45) Date of publication and mention
of the grant of the patent:
**18.02.2026   Bulletin 2026/08**

(21) Application number: **21780111.7**

(22) Date of filing: **29.03.2021**

(51) International Patent Classification (IPC):
**C07C 51/42** *(2006.01)*        **C07C 51/44** *(2006.01)*
**C07C 53/08** *(2006.01)*

(52) Cooperative Patent Classification (CPC):
(C-Sets available)
**C07C 51/42; C07C 51/44;** Y02P 20/10        (Cont.)

(86) International application number:
**PCT/JP2021/013187**

(87) International publication number:
**WO 2021/200785 (07.10.2021 Gazette 2021/40)**

(54) **METHOD FOR PRODUCING PURIFIED ACETIC ACID**

VERFAHREN ZUR HERSTELLUNG VON GEREINIGTER ESSIGSÄURE

PROCÉDÉ DE PRODUCTION D'ACIDE ACÉTIQUE PURIFIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **31.03.2020   JP 2020063081**

(43) Date of publication of application:
**08.02.2023   Bulletin 2023/06**

(73) Proprietor: **Daicel Corporation
Osaka 530-0011 (JP)**

(72) Inventors:
• **TAKEDA, Kazuhito
Tokyo 108-8230 (JP)**
• **FUKUI, Naoyuki
Tokyo 108-8230 (JP)**
• **DEBA, Miduki
Tokyo 108-8230 (JP)**

(74) Representative: **Grünecker Patent- und
Rechtsanwälte
PartG mbB
Leopoldstraße 4
80802 München (DE)**

(56) References cited:
JP-A- 2012 066 240      JP-A- 2013 188 687
JP-A- 2019 131 526      JP-A- H02 111 404
JP-A- H09 151 158       JP-A- H09 151 158

(52) Cooperative Patent Classification (CPC): (Cont.)

C-Sets
**C07C 51/42, C07C 53/08;
C07C 51/44, C07C 53/08**

## Description

Technical Field

The present disclosure relates to a method for producing purified acetic acid. More particularly, the present disclosure relates to a method for producing purified acetic acid, in which the acetic acid is recovered from a mixed solution containing acetic acid, an organic solvent, and water. The present application claims priority from the Japanese patent application No. 2020-063081, filed in Japan on March 31, 2020.

Background Art

Cellulose acetate is used in various applications including cigarette filter tows, fibers, photographic films, and artificial kidneys. As a method of producing cellulose acetate, a method in which an acetylating agent is subjected to a reaction with raw material pulp or cellulose (hereinafter referred to as "raw material pulp or the like") in an acetic acid solvent is known. In this method, an acetic acid aqueous solution (hereinafter referred to as "raw acid") with an acetic acid concentration of 20 to 50 wt.% in which a certain amount of cellulose acetate is dissolved is discharged. Acetic acid is recovered from this raw acid and reused in the production of cellulose acetate.

In the process of recovering acetic acid from the raw acid, the raw acid is first extracted with an organic solvent, the resultant is separated into an extract containing acetic acid, an organic solvent, and water, and a raffinate mainly containing water, and then the extract is distilled to recover acetic acid. In this distillation, it is necessary to separate not only the acetic acid and the organic solvent but also the water. A known distillation method utilizing a boiling point difference is commonly used to separate acetic acid, an organic solvent, and water (Patent Documents 1 and 2).

Patent Document 3 discloses a method for recovering acetic acid including: extracting an acetic acid aqueous solution with an organic solvent; separating the extract by a separation portion including a water-selective permeable separation membrane; supplying a fraction that has passed through the separation portion to the extraction portion; and supplying a fraction that has not passed through the separation portion to a dehydration-desolvation column to continuously recover acetic acid. Patent Document 4 discloses a method for concentrating acetic acid including:

distilling a raw material, which is a mixture of acetic acid and water, in a distillation column to obtain overhead vapor containing acetic acid and water; separating the overhead vapor by a vapor permeable separation membrane to remove water;
condensing the acetic acid vapor by utilizing the acetic acid vapor after removing water to heat the raw material.

Citation List

Patent Document

Patent Document 1: JP 09-048744
Patent Document 2: JP 10-114699
Patent Document 3: JP 02-111404
Patent Document 4: JP 2014-226573

Summary of Invention

Technical Problem

However, the difference in boiling point between acetic acid and water is small, and it is difficult to completely separate water and acetic acid by a distillation method alone. Furthermore, in the separation of acetic acid and water, it is necessary to evaporate and distill water having high latent heat of vaporization, and thus there is an issue that the energy efficiency is not good.

In the method of Patent Document 3, since water is excessively taken, azeotropy is unlikely to occur in the distillation process, thereby causing issues such as the rise of energy requirement and the high flow rate that increases the equipment cost due to the large membrane area. Further, in the method of Patent Document 4, since the vapor at the top of the distillation column is dehydrated by the vapor permeation-separation membrane, the amount of vapor to be used itself is not reduced, and the issue still remains in that a large amount of energy is used. Furthermore, in this method, acetic acid cannot be efficiently separated from a mixed liquid containing acetic acid, an organic solvent, and water. JPH09151158A describes a process for purifying acetic acid, which includes: extractant (isopropyl acetate) added to an aqueous acetic acid solution for extraction, and then azeotropic distillation of the mixture, in order to obtain purified acetic acid.

Therefore, an object of the present disclosure is to provide a method for producing purified acetic acid from a mixed solution containing acetic acid, an organic solvent, and water in an energy-efficient manner.

Solution to Problem

As a result of diligent research to solve the issues described above, the present inventors have studied and found that when purified acetic acid is produced from a mixed solution containing acetic acid, an organic solvent, and water, the membrane separation step that includes separating water in the purified liquid in the distillation column by the separation membrane is incorporated into the process at the subsequent distillation step, thereby minimizing the separation of acetic acid and water in the distillation step and reducing the energy load. The present disclosure has been completed based on these findings.

That is, the present disclosure provides the followings:

[1] A method for producing purified acetic acid from a mixed solution containing acetic acid, an organic solvent, and water, the method including: a distillation step of distilling the mixed solution and separating the mixed solution into a purified liquid rich in acetic acid and a separated liquid rich in an organic solvent; and a membrane separation step of separating the water from the purified liquid by a separation membrane, in which the purified liquid has a water concentration of 4 wt.% or less; wherein the organic solvent is selected from benzene, toluene, n-hexane, cyclohexane, heptane, methyl acetate, ethyl acetate, propyl acetate, chloroform, carbon tetrachloride, 1,2-dimethoxyethane and dimethyl ether, either alone or two or more thereof in combination.

[2] The method for producing purified acetic acid according to [1], in which the purified liquid has an ethyl acetate concentration as measured by gas chromatography of equal to or less than a detection limit;

[3] The method for producing purified acetic acid according to [1] or [2], in which the purified liquid has an organic solvent concentration as measured by gas chromatography of equal to or less than a detection limit;

[4] The method for producing purified acetic acid according to any one of [1] to [3], in which the membrane separation step is performed by bringing the purified liquid into contact with the separation membrane under reduced pressure;

[5] The method for producing purified acetic acid according to any one of [1] to [4] above, in which the separation membrane is one or more zeolite separation membranes selected from the group consisting of A-type, FAU-type, CHA-type, MFI-type, MOR-type, and DDR-type zeolite separation membranes; and

[6] The method for producing purified acetic acid according to any one of [1] to [5], in which the mixed solution is derived from a liquid discharged during a process of producing cellulose acetate.

Note that the organic solvent does not contain acetic acid.

Advantageous Effects of Invention

According to [1] above of the present disclosure, the membrane separation step that includes separating water in the purified liquid by the separation membrane is incorporated to the process at the subsequent distillation step, and thus it is possible efficiently separate water by the subsequent membrane separation step even in a case where the water concentration in the purified liquid in the distillation column reaches 4 wt.%. Thus, an excellent energy-saving effect is exerted such that the energy load required for water separation is greatly reduced in the distillation step.

According to [2] above of the present disclosure, even when the separation efficiency in the distillation step is decreased by reducing the energy load, the ethyl acetate concentration in the purified liquid is equal to less than a detection limit, and thus high-quality purified acetic acid free from ethyl acetate can be obtained by subjecting the purified liquid to the membrane separation step.

According to [3] above of the present disclosure, even when the separation efficiency in the distillation step is decreased by reducing the energy load, the detectable level of concentration of the organic solvent in the purified liquid is equal to less than a detection limit, and thus high-quality purified acetic acid free from contamination with the organic solvent can be obtained by subjecting the purified liquid to the membrane separation step.

According to [4] above of the present disclosure, separation between acetic acid and water can be achieved by the difference in pressure between the inlet side and the permeate side of the separation membrane remaining in a liquid state. Therefore, when water is separated from acetic acid, it is not necessary to vaporize the purified liquid by heating, and thus an excellent vapor-saving effect is given and the energy load can be greatly reduced.

According to [5] above of the present disclosure, a specific zeolite separation membrane is used, and thus an effect is exerted in which the operating conditions with excellent dehydration efficiency can be adjusted depending on the composition of the raw material.

According to [6] above of the present disclosure, it is possible to further improve the separation efficiency of acetic acid contained in the liquid (i.e., raw acid) discharged during the process of producing cellulose acetate. These result in an excellent vapor-saving effect in the process of recovering acetic acid, and the energy load can be greatly reduced. Recycling of the purified acetic acid obtained by the method of the present invention during the process of producing cellulose acetate can also contribute to a reduction in the production cost of cellulose acetate.

Brief Description of Drawings

FIG. 1 is a schematic flow chart illustrating an example of a method for producing purified acetic acid of the present disclosure.
FIG. 2 is a schematic flow chart illustrating an apparatus for testing performance of a separation membrane in a preliminary test.
FIG. 3 is a graph showing a relationship between an acetic acid concentration $X11$ and a permeation rate Q in a model purified liquid in the preliminary test.
FIG. 4 is a graph showing a relationship between the acetic acid concentration $X11$ in the model purified liquid in the preliminary test and an acetic acid concentration $X21$ in a permeated liquid.
FIG. 5 is a graph showing a relationship between the acetic acid concentration $X11$ in the model purified liquid in the preliminary test and a permeability coefficient K.
FIG. 6 is a graph showing a relationship between the acetic acid concentration $X11$ in the model purified liquid in the preliminary test and a separation coefficient $\alpha$.
FIG. 7 is a graph showing a vapor use rate (when the reference is 100) of a distillation column 3 in a case where the water concentration in the purified liquid in the distillation column 3 in Example 1 is varied from 0 to 5 wt.%.
FIG. 8 is a graph showing the acetic acid concentration (wt. ppm, indicated by a rhombus) in an upper layer liquid in a decanter and the ethyl acetate concentration (wt. ppm, indicated by a triangle) in the purified liquid in the distillation column 3, in a case where the water concentration in the purified liquid in the distillation column 3 in Example 1 is varied from 0 to 5 wt.%.

Description of Embodiments

Method for Producing Purified Acetic Acid

As a raw material to be used in the present disclosure, raw acid is commonly used, but the raw material is not limited to the raw acid. An acetic acid-containing solution containing at least acetic acid (e.g., an aqueous solution including acetic acid, a mixture of acetic acid, water, and various solvents, etc.) may be used. In the acetic acid-containing solution containing at least acetic acid, the total content of acetic acid and water is, for example, 50 wt.% or greater, preferably 80 wt.% or greater, more preferably 90 wt.% or greater, and still more preferably 95 wt.% or greater.

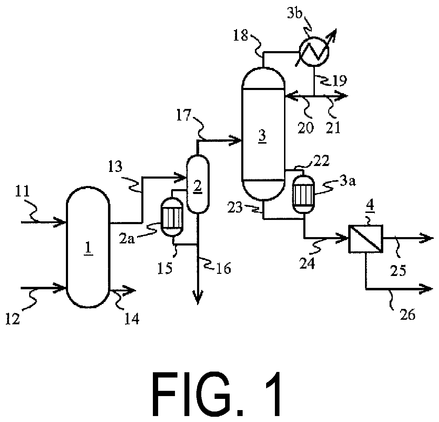

Hereinafter, an example of an embodiment using raw acid as a raw material will be described with reference to FIG. 1. FIG. 1 is a schematic view illustrating a production flow of purified acetic acid of the present disclosure. A purified acetic acid production apparatus according to this production flow includes an extraction column 1, an evaporator 2, a distillation column 3, a separation membrane 4, reboilers 2a and 3a, a capacitor 3b, and lines 11 to 26. Note that in Examples described below, an acetic acid-containing solution containing at least acetic acid other than raw acid can be used in place of the raw acid.

Extraction Step

Extraction step is a process of bringing raw acid into contact with an extractant and separating the raw acid into an extractant liquid phase mainly containing acetic acid, the extractant, and a small amount of water, and an extracted residual phase mainly containing water. In FIG. 1, the raw acid is introduced into the extraction column 1 through the line 11. The acetic acid concentration in the raw acid is usually from 20 to 50 wt.%. This raw acid includes water, cellulose acetate, and the like, in addition to acetic acid. Impurities in the raw acid may be removed in advance with diatomaceous earth, activated carbon, or the like.

The extractant is introduced from the line 12 into the extraction column 1. The extractant is preferably a solvent that is easily separated from acetic acid in the subsequent distillation step and has a boiling point (azeotropic temperature in the case of azeotropic with water) lower than the boiling point of acetic acid, for example, a solvent having a boiling point (azeotropic temperature in the case of azeotropic with water) lower than the boiling point of acetic acid by equal to or higher than 10°C. The solvent is selected from benzene, toluene, n-hexane, cyclohexane, heptane, methyl acetate, ethyl acetate, propyl acetate, chloroform, carbon tetrachloride, 1,2-dimethoxyethane and dimethylether. One kind of the extractant can be used alone or two or more kinds in combination.

The amount of the extractant to be used is, for example, from about 1 to 2.5 times (volume ratio), and preferably from about 1.5 to 1.7 times (volume ratio) the amount of the raw acid.

The extraction column 1 may be a commonly used type of column, an example of which includes a mixer-settler type extraction column, a perforated-plate type extractor, a packed-column type extractor, a baffle column type extractor, a vibrating perforated-plate type extractor, a stirring and mixing type extractor, a pulsating packed type extractor, or a centrifugal extraction type extractor. The extraction operation in the extraction column 1 can be performed intermittently or continuously. In a case where the extraction effect is insufficient, repeated extraction operations can also be performed.

The contact temperature between raw acid and the extractant is, for example, in a range from 20 to 60°C, and preferably in a range from 30 to 50°C. The contact time is, for example, approximately from 0.1 to 10 hours.

The contact between raw acid and the extractant can be performed under normal pressure, under pressure, or under reduced pressure.

The extractant liquid phase obtained in this step mainly includes acetic acid and the extractant, and the water concentration is, for example, 15 wt.% or less, and preferably 10 wt.% or less. The cellulose acetate concentration is 200 wt. ppm or less, and preferably 150 wt. ppm or less. Meanwhile, the extracted residual phase mainly contains water and soluble cellulose acetate, and the acetic acid concentration is, for example, 0.5 wt.% or less, and preferably 0.1 wt.% or less.

Evaporation Step

Evaporation step is a process of introducing the extractant liquid phase into the evaporator 2 from the line 13, and evaporating the extractant liquid phase to cause separation into vapor including acetic acid, the extractant, and water and an evaporated residual liquid containing soluble cellulose acetate. In this step, the separated soluble cellulose acetate is discharged to the outside of the system through the line 16. In this step, the cellulose acetate dissolved in the extractant liquid phase can be separated and removed.

In this step, it is sufficient that the extractant liquid phase can be separated into vapor including acetic acid, the extractant, and water and an evaporated residual liquid containing soluble cellulose acetate. For example, the separation can be achieved by subjecting the extractant liquid phase to a heating treatment. The temperature of heating treatment is, for example, from 80 to 100°C. The heating treatment can be performed under normal, pressurized, or reduced pressure. It may include a single step, or may include a combination of multiple steps. A known evaporator can be used as the evaporator 2, and an evaporation tank in the form of distillation column may also be used.

The vapor mainly contains acetic acid, the extractant, and water. The acetic acid concentration in vapor is, for example, 10 wt.% or greater, and preferably 15 wt.% or greater. The soluble cellulose acetate concentration is 50 wt. ppm or less, and preferably 20 wt. ppm or less. The water concentration is, for example, 10 wt.% or less, and preferably 8 wt.% or less.

Distillation Step

The distillation step is a process of separating acetic acid, the extractant, and water, utilizing a boiling point difference, and the extractant and water are distilled off to obtain acetic acid as a purified liquid. In the distillation step, the extractant liquid phase obtained in the extraction step may be fed into the distillation column 3 in liquid phase (not illustrated), or the vapor obtained in the evaporating may be fed into the distillation column 3 from the line 17.

When the extractant liquid phase or the vapor is fed into the distillation column 3, large portions of the extractant and water contained in the extractant liquid phase or vapor pass through the line 18 from the column top and are cooled in a cooler 3b, being introduced into a tank (decanter) (not illustrated). The upper layer liquid of the liquid introduced into the tank mainly contains the extractant, and is partially refluxed into the distillation column 3. The rest of the liquid in the tank is discharged outside the system. This discharged liquid can be reused again as an extractant to be introduced into the extraction column 1. The lower layer liquid in the tank mainly contains water, and is discharged outside the system. Meanwhile, acetic acid is obtained from the column bottom of the distillation column 3 through the line 23.

Examples of the distillation column 3 include a plate column or a packed column. The column top temperature is, for example, from 20 to 120°C, preferably from 30 to 100°C, and more preferably from 40 to 80°C. The pressure in the distillation column 3 is, for example, from 0.1 to 0.5 MPa (absolute pressure). Further, the distillation can also be performed under reduced pressure. The distilling may be comprised of a single step, or may be comprised of a combination of steps.

The number of theoretical plates and the reflux ratio in the distillation column 3 can be set as appropriate in consideration of the separation efficiency and the vapor cost. In the present invention, since the purified liquid in the distillation column 3 is subjected to the membrane separation step described below, water contained in the purified liquid is separated, and thus the liquid to be supplied to the distillation column 3 does not need to be completely distilled the water contained in the vapor from the top of the column. Consequently, this allows the number of actual plates and the reflux ratio in the distillation column 3 to be greatly reduced as compared with those in the related art, thereby achieving saving of energy.

After undergoing the distillation step, acetic acid containing a trace or small amount of water as purified liquid is obtained from the line 24. The lower limit of the acetic acid concentration in the purified liquid is, for example, 96 wt.%, preferably 96.5 wt.%, and more preferably 97 wt.%. In the present invention, the purified liquid is subjected to the membrane separation step described below, and thus it is possible to set the acetic acid concentration in the purified liquid to a concentration lower than the known concentration.

The water concentration in the purified liquid is, for example, 4 wt.% or less. In the present invention, since acetic acid and water are separated by the membrane separation step described below, it is possible to set conditions in which the water concentration in acetic acid after being subjected to the distillation step is reduced to 4 wt.% or less. This makes it possible to minimize the separation of acetic acid and water in the distillation step, an excellent vapor-saving effect is given, and the energy load can be greatly reduced.

The concentration of an organic solvent (e.g., an ester such as ethyl acetate, an aromatic hydrocarbon such as benzene, etc.) in the purified liquid is, for example, 0.1 wt. ppm or less, preferably 0.05 wt. ppm or less, and more preferably 0.01 wt. ppm or less. Preferably, the concentration of each of the organic solvents in the purified liquid is equal to or less than the detection limit. More preferably, the concentration of all the organic solvents is equal to or less than the detection limit. The term "detection limit" used herein means that the concentration of an organic solvent in the purified liquid measured by the following measurement method is equal to or less than the detection limit.

Method for Measuring Organic Solvent

The purified liquid to be measured is analyzed by gas chromatography (GC-2010, Shimazu Corporation), and the concentration of an organic solvent is measured by quantifying the organic solvent in the purified liquid (detector: FID).

The column top distillate is mainly a solution rich in an organic solvent such as ethyl acetate or benzene. The acetic acid concentration in the column top distillate is, for example, 0.02 wt.% or less, preferably 0.01 wt.% or less, and more preferably 0.005 wt.% or less. The acetic acid concentration in the column top distillate can be controlled by adjusting the reflux ratio of the distillation column 3.

When the column top distillate is divided into an organic phase and an aqueous phase, the acetic acid concentration in the organic phase is, for example, 0.05 wt.% or less, preferably 0.02 wt.% or less, more preferably 0.01 wt.% or less, and still more preferably 0.005 wt.% or less.

Membrane Separation Step

The membrane separation step is a process of separating water from the purified liquid by the separation membrane 4. The separating by the membrane can be performed by introducing the purified liquid into the separation membrane 4 from the line 24.

In the membrane separation step, for example, the purified liquid is directly brought into contact with the separation membrane 4, causing water to permeate selectively from a mixture of acetic acid and water. This method is referred to as pervaporation. In FIG. 1, the purified liquid is supplied to the inlet side of the separation membrane 4, the permeate side is depressurized, and thus a transmembrane pressure difference is generated, thereby obtaining a driving force for moving the purified liquid from the inlet side to the permeate side. The separation is caused by a difference in permeation rate between acetic acid and water.

The method for depressurizing the permeate side is not particularly limited, and the permeate side can be depressurized by condensing water passing through the permeate side of the separation membrane 4 with a cooling source (not illustrated). In this case, there is an advantage that a partial pressure difference, which is a driving force for causing water to permeate the membrane, is secured, whereby the area of the membrane can be reduced and electric power such as a vacuum pump becomes unnecessary.

The pressure on the permeate side of the separation membrane 4 is, for example, in a range of 100 to 400 Pa, and preferably in a range of 200 to 300 Pa.

The temperature at which the purified liquid is fed into the separation membrane 4 is not particularly limited, and is usually from 20 to 160°C, preferably from 50 to 150°C, more preferably from 70 to 145°C, and still more preferably from 80 to 140°C. As for the flow rate for feeding the purified liquid into the separation membrane 4, for example, in the case of using a flow rate of 1 m/hr as an indicator, the flow rate per one is approximately 0.6 $m^3$/hr.

The area of the separation membrane 4 is not particularly limited, and is, for example, from 800 to 2500 $m^2$.

Water is discharged to the permeate side by the line 26, and acetic acid as a non-permeable component is separated by the line 25. The acetic acid concentration in the recovered acetic acid is determined by the required quality, and is, for example, 98 wt.% or greater, preferably 99 wt.% or greater, and more preferably 99.5 wt.% or greater. The acetic acid concentration in the permeated water is, for example, 1 wt.% or less.

The performance of the separation membrane 4 is not particularly limited, and can be determined as appropriate depending on the acetic acid concentration and water concentration in the purified liquid to be introduced.

The separation coefficient α of the separation membrane 4 with respect to water is, for example, 1000 or greater, and preferably 2000 or greater. The separation coefficient α herein can be obtained from the following Formula (1):

$$\text{Separation coefficient } \alpha = ((100 - X21)/X21)/((100 - X11)/X11) \ (1)$$

where X21 indicates an acetic acid concentration in the permeated liquid (wt.%), and X11 indicates an acetic acid concentration in the purified liquid (wt.%).

The water permeation rate Q of the separation membrane 4 is, for example, 0.5 kg/m$^2$·hr or greater (e.g., from 0.5 to 5 kg/m$^2$·hr), preferably 0.8 kg/m$^2$·hr or greater (e.g., from 0.8 to 3 kg/m$^2$·hr), more preferably 0.9 kg/m$^2$·hr or greater (e.g., from 0.9 to 2.5 kg/m$^2$·hr) when the water content in the purified liquid is 4 wt.%, and is, for example, 0.01 kg/m$^2$·hr or greater (e.g., from 0.01 to 0.8 kg/m$^2$·hr), preferably 0.02 kg/m$^2$·hr or greater (e.g., from 0.02 to 0.5 kg/m$^2$·hr), more preferably 0.025 kg/m$^2$·hr or greater (e.g., from 0.025 to 0.4 kg/m$^2$·hr) when the water content in the purified liquid is 0.5 wt.%.

The permeability coefficient K of the separation membrane 4 is, for example, 0.00001 kg/m$^2$·hr·Pa or greater (e.g., from 0.00001 to 0.00008 kg/m$^2$·hr·Pa), preferably 0.00002 kg/m$^2$·hr·Pa or greater (e.g., from 0.00002 to 0.00006 kg/m$^2$·hr·Pa), and more preferably 0.000025 kg/m$^2$·hr·Pa or greater (e.g., from 0.000025 to 0.00005 kg/m$^2$·hr·Pa). The permeability coefficient K herein can be obtained from the following Formula (2):

$$\text{Permeation rate } K = W/A/\Delta P \ (2)$$

where W indicates a permeated liquid volume (kg/hr), A indicates a membrane surface area (m$^2$), ΔP indicates a water partial pressure difference (Pa).

Examples of the material of the separation membrane 4 can include a polymer membrane, a ceramic membrane, or a zeolite membrane. Among these materials, a zeolite membrane is preferable from the viewpoint of hydrophilicity, permeation rate, water separation selectivity, and acid resistance. The zeolite membrane is not particularly limited, and can be selected as appropriate. For example, preferred are one or more zeolite separation membranes selected from the group consisting of A-type, FAU-type, CHA-type, MFI-type, MOR-type, and DDR-type zeolite separation membranes, and the CHA-type is more preferred from the viewpoint of being excellent in hydrophilicity, permeation rate, water separation selectivity, and acid resistance, and having durability. The CHA-type zeolite membrane can be sieved according to the difference between the molecular size of acetic acid and the molecular size of water by a size exclusion method, and thus the membrane has high separation performance.

The configuration of the separation membrane 4 (micropore size, shape, porosity and non-porosity, presence or absence of a support, and the like) is not particularly limited, and can be determined as appropriate in consideration of the temperature of feed, the flow rate of feed, the separation coefficient α, the permeation rate Q, the permeability coefficient K, and the like. For example, the CHA-type zeolite membrane has a three-dimensional pore structure, and has a high diffusion rate, high hydrophilicity, and a large adsorption amount of water. Accordingly, it is possible to obtain a high permeation flux (Flux). The separation membrane 4 may be used alone, or may be used in the form of a multitubular membrane, a so-called separation membrane module.

The separation membrane 4 may be a commercially available product. Examples thereof can include a CHA-type zeolite membrane (trade name "HZM-4", Hitachi Zosen Corporation).

After undergoing the membrane separation step, acetic acid and water in the purified liquid can be efficiently separated. Thus, highly purified acetic acid (hereinafter referred to as "purified acetic acid") can be recovered by the line 26. According to the method of the present invention, when separating water from acetic acid, the distillation step is combined with the membrane separation step, so the amount of heat injected into the distillation column 3 can be greatly reduced (vapor-saving effect). Accordingly, this makes it possible to achieve saving of energy, and to construct an industrially efficient method for producing purified acetic acid. Further, after the membrane separation step, the purified acetic acid can be incorporated in the process of producing cellulose acetate, and can also be fed into a reactor together with a raw material pulp or the like. Therefore, a reduction in the production cost of cellulose acetate can be achieved.

Each aspect disclosed in the present specification can be combined with any other feature disclosed herein. The present disclosure is not limited by the embodiments and is limited only by the claims.

Examples

Hereinafter, the present invention will be specifically described with reference to examples.

Preliminary Test

To examine the performance of the separation membrane 4, the following performance testing of the separation membrane was performed using a single separation membrane module.

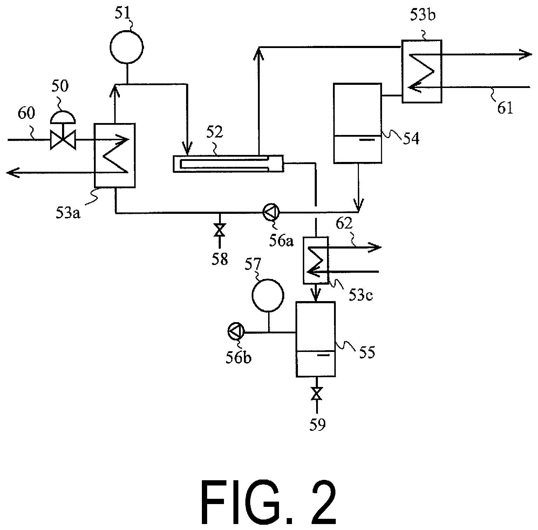

The model purified liquid was used to evaluate the performance of a single module of a CHA-type zeolite membrane as the separation membrane 4. The apparatus used for testing the performance is illustrated in FIG. 2. In FIG. 2, the apparatus for testing performance includes a TCV 50, a TIC 51, a membrane module 52 (separation membrane), heat exchangers 53a, 53b, and 53c, a tank (purified liquid) 54, a tank (permeated liquid) 55, pumps 56a and 56b, a pressure transmitter 57, a purified liquid sample 58, a permeated liquid sample 59, vapor 60, cooling water 61, and cold water 62.

Test Method

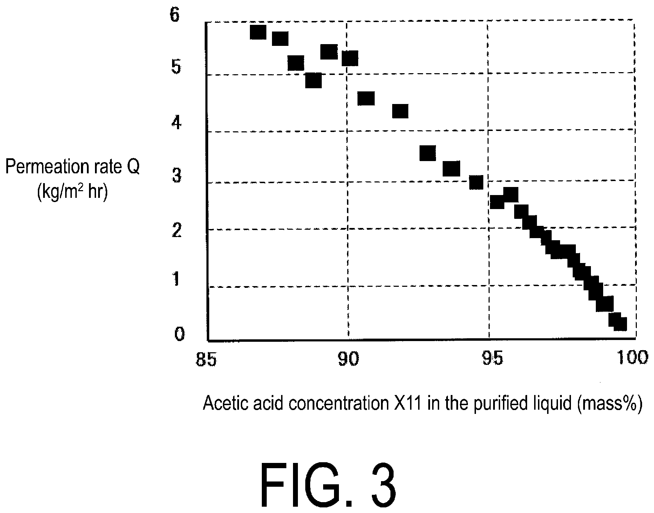

After feeding the model purified liquid into the tank 54, the vapor 60, the cooling water 61, and the cold water 62 were supplied, and the operation was started under the conditions below. Thereafter, the vacuum pump was operated to start permeation. The concentration in the acetic acid concentrated liquid, the acetic acid concentration in the permeated liquid, and the amount of the permeated liquid were measured every 1 hour from the start of the test. Based on these measured values, the acetic acid concentration $X21$, the permeation rate Q, the separation coefficient $\alpha$, and the permeability coefficient K were evaluated in the permeated liquid from the start of the test until 258 hours after the start of the test. The results are shown in the graphs of FIGS. 3 to 6.

Test Conditions

· Model purified liquid: acetic acid concentration 87 wt.% (1 specimen in total) · Data collection: 30 times in total
· Membrane module: trade name "HZM-4", body diameter: 16 mm × length 60 mm, membrane surface area: 0.05 m$^2$, CHA-type zeolite membrane, manufactured by Hitachi Zosen Corporation
· Temperature of feed: 130°C
· Flow rate of feed: 120 kg/hr
· Pressure in a space at the permeate side of the separation membrane: 100 to 200 Pa

FIG. 3 is a graph showing the relationship between the acetic acid concentration in the model purified liquid and the permeation rate Q. The permeation rate Q tended to decrease as the acetic acid concentration increased, indicating that the amount of water molecules permeating the separation membrane decreased. The results were as expected.

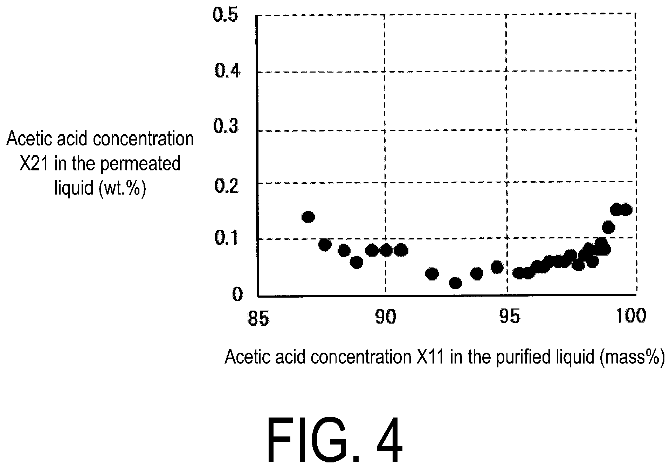

FIG. 4 is a graph showing the relationship between the acetic acid concentration in the model purified liquid and the acetic acid concentration $X21$ in the permeated liquid. It was confirmed that the acetic acid concentration $X21$ in the permeated liquid showed a stable value (about 0.08 wt.%) even when the acetic acid concentration in the model purified liquid varied, and dehydration was stably performed.

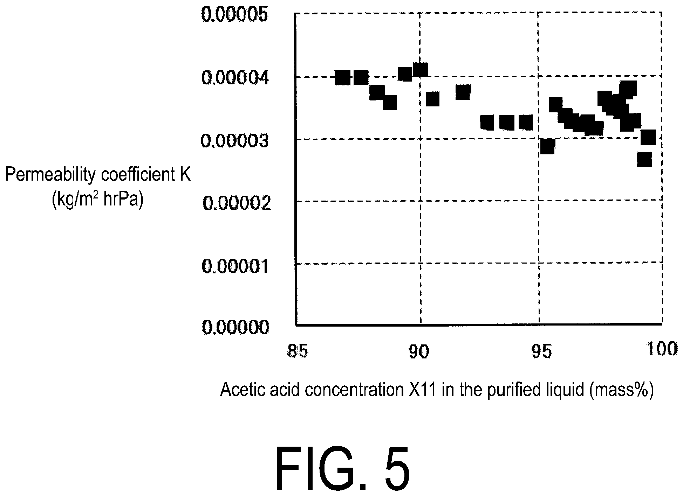

FIG. 5 is a graph showing the relationship between the acetic acid concentration in the model purified liquid and the permeability coefficient K. It was confirmed that the permeability coefficient K showed a stable value (in a range of 0.00003 to 0.00004) even when the acetic acid concentration in the model purified liquid varied, and dehydration was stably performed.

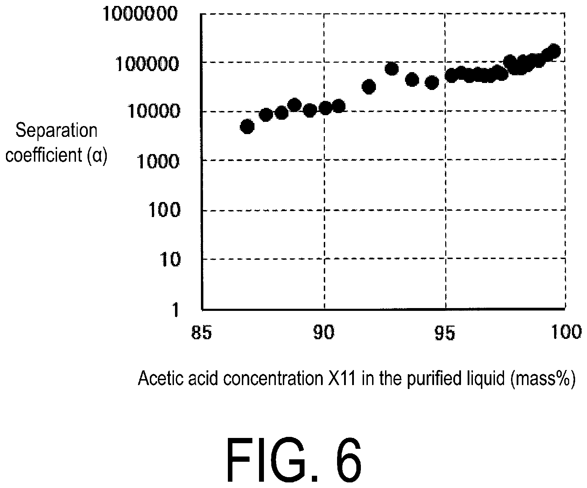

FIG. 6 is a graph showing the relationship between the acetic acid concentration $X11$ in the model purified liquid and the separation coefficient $\alpha$. It was confirmed that the separation coefficient $\alpha$ showed a constant value in a stable range even when the acetic acid concentration in the model purified liquid varied, and stable membrane separation was performed.

From the performance test results of the membrane module described above, it was confirmed that the membrane separation step by the separation membrane was introduced, thereby allowing for industrially efficient separation between acetic acid and water.

Example 1

A test was performed to demonstrate the energy-saving effect in a case where the method of the present invention was used in the acetic acid recovery plant, based on the results of the performance test of the separation membrane. The test conditions are as follows.

Distillation Step

· Composition fed to the distillation column 3

Acetic acid: 17 wt.%
Water: 8 wt.%
Ethyl acetate: 56 wt.%
Benzene: 19 wt.%

· Temperature of feed: 91.7°C
· Column top pressure: 1 atm
· The number of plates of the distillation column 3: 29 plates
· Position of feed: at the 10th plate from the top
· Feed of liquid and feed of vapor: feed of vapor
· Reflux ratio: 0.74

Membrane Separation Step

Conditions

· Material of separation membrane: CHA-type zeolite membrane
· Temperature of feed: 130°C
· Concentration of water fed: 4 wt.%
· Flow rate of feed: 10.1 t/hr
· Pressure in a space at the permeate side of the separation membrane: 1 kPa
· Concentration of water at the outlet: 0.5 wt.%
· Outlet flow rate: 9.74 t/hr
· Water concentration in the permeated liquid: 99 wt.%
· Amount of permeated liquid: 0.36 t/hr

This result showed that the separation coefficient $\alpha$ was 2376, which was sufficient as the performance of the membrane.

Next, the water concentration in the purified liquid in the distillation column was varied from 0 to 5 wt.% by changing the reflux ratio of the distillation column. Then, it was examined how the vapor use rate of the distillation column, the acetic acid concentration in the upper layer liquid in the overhead vapor condensate (stored in the decanter), and the ethyl acetate concentration in the purified liquid in the distillation column were changed.

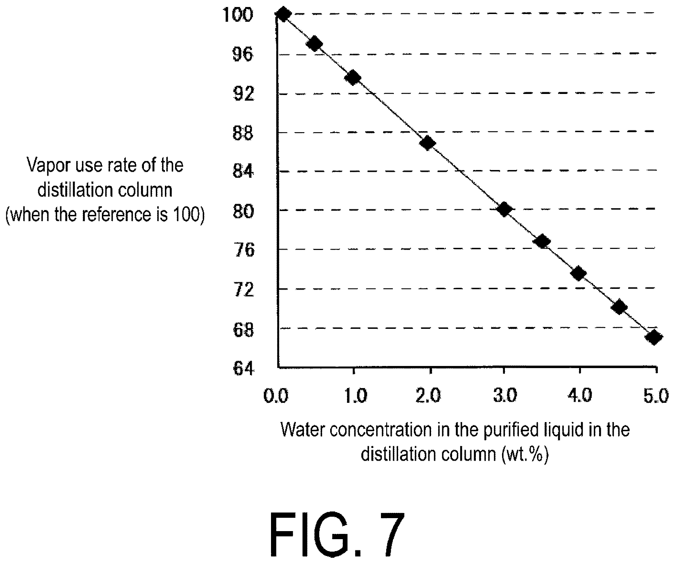

FIG. 7 is a graph showing the vapor use rate of the distillation column 3 in a case where the water concentration in the purified liquid in the distillation column is varied from 0 to 5 wt.% by changing the reflux ratio of the distillation column. In the known method for producing purified acetic acid, the water concentration in the purified liquid in the distillation column 3 was set to as close as possible to 0 wt.% (100 based on the vapor use rate in this case). From this result, it can be seen that the water concentration in the purified liquid is reduced to 4 wt.% or less, and as a result, the vapor use rate of the distillation column 3 can be reduced to about 74.

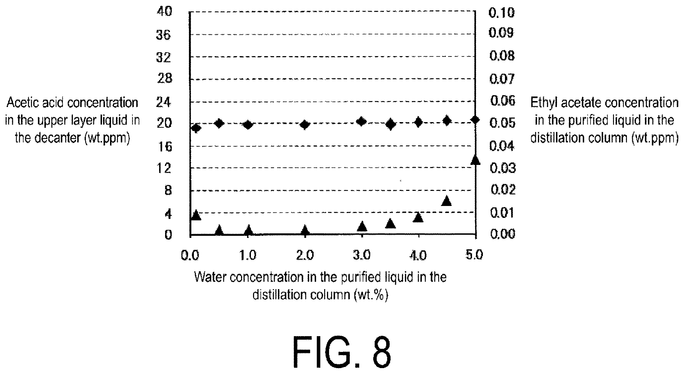

FIG. 8 is a graph showing the acetic acid concentration (wt. ppm, indicated by a rhombus) in an upper layer liquid in a decanter and the ethyl acetate concentration (wt. ppm, indicated by a triangle) in the purified liquid in the distillation column, in a case where the water concentration in the purified liquid in the distillation column is varied from 0 to 5 wt.% by changing the reflux ratio in the distillation column. Lowering of the vapor use rate of the distillation column 3 leads to lowering of the separation efficiency between acetic acid and the organic solvent. From this result, it was confirmed that even when the water concentration in the purified liquid in the distillation column was reduced to 4 wt.%, the acetic acid concentration in the upper layer liquid in the decanter did not change, and the ethyl acetate concentration in the purified liquid was equal to or less than the detection limit (0.01 ppm or less). Note that the value of concentration of ethyl acetate in the purified liquid when the concentration of water in the purified liquid in the distillation column is changed from 0 wt.% to 4 wt.% is a value obtained by the simulation.

Based on the results described above, the membrane separation step that includes separating water in the purified liquid by the separation membrane is incorporated into the process at the subsequent distillation step, and thus it is possible efficiently separate water by the membrane separation step even in a case where the water concentration in the purified liquid in the distillation column is 4 wt.% at a maximum. Therefore, it was confirmed that a significant vapor-saving effect was obtained in the distillation step, and an excellent energy-saving effect was exerted.

In the embodiment shown in FIG. 1, an aspect is illustrated in which one distillation column 3 and one separation membrane 4 are disposed, but the number of each of the distillation column and the separation membrane is not limited to one. The number of the distillation column 3 or the separation membrane 4 can be determined as appropriate depending on the required concentration of the purified acetic acid. In addition, the present invention is not limited to the embodiments described above, but variations and modifications within the scope where the object of the present invention can be achieved are included in the present invention.

As a summary of the above, configurations and variations of the present disclosure are described below.

[1] A method for producing purified acetic acid from a mixed solution containing acetic acid, an organic solvent, and water, the method including: a distillation step of distilling the mixed solution and separating the mixed solution into a purified liquid rich in acetic acid and a separated liquid rich in an organic solvent; and a membrane separation step of separating the water from the purified liquid by a separation membrane, in which the purified liquid has a water concentration of 4 wt.% or less;

[2] The method for producing purified acetic acid according to [1] above, in which the mixed solution is derived from a liquid discharged during a process of producing cellulose acetate.

[3] A method for producing purified acetic acid, the method including: an extraction step (A) of bringing an acetic acid-containing solution containing at least acetic acid (or an acetic acid-containing solution discharged during a process of producing cellulose acetate) into contact with a extractant and separating the acetic acid-containing solution into an extractant liquid phase mainly containing acetic acid, the extractant, and a small amount of water, and an extracted residual phase mainly containing water and soluble cellulose acetate; an evaporation step (B) of, by introducing at least part of the extractant liquid phase into an evaporator, evaporating and separating the extractant liquid phase into vapor containing acetic acid, the extractant, and water, and an evaporated residual liquid containing soluble cellulose acetate; a distillation step (C) of supplying the vapor (or the extractant liquid phase and the vapor) to a distillation column and separating the vapor into a distillate containing the extractant and water and a purified liquid containing acetic acid; and a membrane separation step (D) of separating the water from the purified liquid containing acetic acid by a separation membrane to obtain purified acetic acid.

[4] A method for producing purified acetic acid, the method including: an extraction step (A) of bringing an acetic acid-containing solution containing at least acetic acid (or an acetic acid-containing solution discharged during a process of producing cellulose acetate) into contact with a extractant and separating the acetic acid-containing solution into an extractant liquid phase mainly containing acetic acid, the extractant, and a small amount of water, and an extracted residual phase mainly containing water and soluble cellulose acetate; a distillation step (C) of supplying the extractant liquid phase to a distillation column and separating the extractant liquid phase into a distillate containing the extractant and water and a purified liquid containing acetic acid; and a membrane separation step (D) of separating water from the purified liquid containing acetic acid by a separation membrane to obtain purified acetic acid.

[5] The method for producing purified acetic acid according to any one of [1] to [4] above, in which the purified liquid has an ethyl acetate concentration as measured by gas chromatography of 0.1 wt. ppm or less (or 0.05 wt. ppm or less, 0.01 wt. ppm or less, or equal to or less than a detection limit).

[6] The method for producing purified acetic acid according to any one of [1] to [5] above, in which the purified liquid has a benzene concentration as measured by gas chromatography of 0.1 wt. ppm or less (or 0.05 wt. ppm or less, 0.01 wt. ppm or less, or equal to or less than a detection limit).

[7] The method for producing purified acetic acid according to any one of [1] to [6] above, in which the purified liquid has an organic solvent concentration as measured by gas chromatography of equal to or less than a detection limit.

[8] The method for producing purified acetic acid according to any one of [1] to [7] above, in which the membrane separation step is performed by bringing the purified liquid into contact with the separation membrane under reduced pressure.

[9] The method for producing purified acetic acid according to any one of [1] to [8] above, in which the separation membrane is one or more zeolite separation membranes selected from the group consisting of A-type, FAU-type, CHA-type, MFI-type, MOR-type, and DDR-type zeolite separation membranes.

Industrial Applicability

The method for producing purified acetic acid of the present disclosure is appropriate as a method for producing purified acetic acid from a mixed solution containing acetic acid, an organic solvent, and water in an energy-efficient manner.

Reference Signs List

1 Extraction column
2 Evaporator
2a, 3a Reboiler
3b Capacitor
3 Distillation column
4 Separation membrane
11 to 26 Line
50 TCV
51 TIC (temperature indicating controller)
52 Membrane module (separation membrane)
53a, 53b, 53c Heat exchanger
54 Tank (purified liquid)
55 Tank (permeated liquid)
56a, 56b Pump
57 Pressure transmitter
58 Purified liquid sample
59 Permeated liquid sample
60 Vapor
61 Cooling water
62 Cold water

**Claims**

1. A method for producing purified acetic acid from a mixed solution containing acetic acid, an organic solvent and water,

   wherein the organic solvent is selected from benzene, toluene, n-hexane, cyclohexane, heptane, methyl acetate, ethyl acetate, propyl acetate, chloroform, carbon tetrachloride, 1,2-dimethoxyethane and dimethyl ether, either alone or two or more thereof in combination,
   the method comprising:

   distilling the mixed solution and separating the mixed solution into a purified liquid rich in acetic acid and a separated liquid rich in an organic solvent; and
   separating the water from the purified liquid by a separation membrane (4),
   wherein the purified liquid has a water concentration of 4 wt.% or less.

2. The method for producing purified acetic acid according to claim 1, wherein the purified liquid has an ethyl acetate concentration as measured by gas chromatography of equal to or less than a detection limit.

3. The method for producing purified acetic acid according to claim 1 or 2, wherein the purified liquid has an organic solvent concentration as measured by gas chromatography of equal to or less than a detection limit.

4. The method for producing purified acetic acid according to any one of claims 1 to 3, wherein the separation is performed by bringing the purified liquid into contact with the separation membrane (4) under reduced pressure.

5. The method for producing purified acetic acid according to any one of claims 1 to 4, wherein the separation membrane (4) is one or more zeolite separation membrane(s) (4) selected from the group consisting of A-type, FAU-type, CHA-type, MFI-type, MOR-type, and DDR-type zeolite separation membranes (4).

6. The method for producing purified acetic acid according to any one of claims 1 to 5, wherein the mixed solution is derived from a liquid discharged during a process of producing cellulose acetate.

**Patentansprüche**

1. Verfahren zum Herstellen gereinigter Essigsäure aus einer gemischten Lösung, die Essigsäure, ein organisches Lösungsmittel und Wasser enthält,

   wobei das organische Lösungsmittel aus Benzen, Toluen, n-Hexan, Cyclohexan, Heptan, Methylacetat, Ethylacetat, Propylacetat, Chloroform, Tetrachlorkohlenstoff, Ethylenglycoldimethylether sowie Dimethylether, entweder einem oder zwei oder mehr dieser Stoffe in Kombination ausgewählt wird,
   und das Verfahren umfasst:

   Destillieren der gemischten Lösung und Trennen der gemischten Lösung in eine gereinigte Flüssigkeit, die reich an Essigsäure ist, und eine getrennte Flüssigkeit, die reich an einem organischen Lösungsmittel ist; sowie
   Abscheiden des Wassers aus der gereinigten Flüssigkeit mittels einer Trennmembran (4),
   wobei die gereinigte Flüssigkeit eine Wasserkonzentration von 4 Gew.-% oder weniger hat.

2. Verfahren zum Herstellen gereinigter Essigsäure nach Anspruch 1, wobei die gereinigte Flüssigkeit eine mittels Gaschromatographie gemessene Ethylacetat-Konzentration hat, die auf oder unter einer Erfassungsgrenze liegt.

3. Verfahren zum Herstellen gereinigter Essigsäure nach Anspruch 1 oder 2, wobei die gereinigte Flüssigkeit eine mittels Gaschromatographie gemessene Konzentration an organischem Lösungsmittel hat, die auf oder unter einer Erfassungsgrenze liegt.

4. Verfahren zum Herstellen gereinigter Essigsäure nach einem der Ansprüche 1 bis 3, wobei die Trennung durchgeführt wird, indem die gereinigte Flüssigkeit unter Unterdruck in Kontakt mit der Trennmembran (4) gebracht wird.

5. Verfahren zum Herstellen gereinigter Essigsäure nach einem der Ansprüche 1 bis 4, wobei es sich bei der Trennmembran (4) um eine oder mehrere Zeolith-Trennmembran/en (4) handelt, die aus der Gruppe ausgewählt wird/-werden, die aus A-Typ-, FAU-Typ-, CHA-Typ-, MFI-Typ, MOR-Typ- und DDR-Typ-Zeolith-Trennmembranen (4) besteht.

6. Verfahren zum Herstellen gereinigter Essigsäure nach einem der Ansprüche 1 bis 5, wobei die gemischte Lösung aus einer Flüssigkeit gewonnen wird, die während eines Prozesses zum Herstellen von Celluloseacetat abgeleitet wird.

**Revendications**

1. Procédé de production d'acide acétique purifié à partir d'une solution mixte contenant de l'acide acétique, un solvant organique et de l'eau,

   dans lequel le solvant organique est choisi parmi le benzène, le toluène, le n-hexane, le cyclohexane, l'heptane, l'acétate de méthyle, l'acétate d'éthyle, l'acétate de propyle, le chloroforme, le tétrachlorométhane, le 1,2-diméthoxyéthane et l'éther diméthylique, soit seuls, soit à raison de deux ou plus parmi ceux-ci en association, le procédé comprenant :

   la distillation de la solution mixte et la séparation de la solution mixte en un liquide purifié riche en acide acétique et un liquide séparé riche en solvant organique ; et
   la séparation de l'eau à partir du liquide purifié à l'aide d'une membrane de séparation (4),
   dans lequel le liquide purifié a une concentration en eau de 4 % en poids ou moins.

2. Procédé de production d'acide acétique purifié selon la revendication 1, dans lequel le liquide purifié a une concentration en acétate d'éthyle, telle que mesurée par chromatographie en phase gazeuse, inférieure ou égale à une limite de détection.

3. Procédé de production d'acide acétique purifié selon la revendication 1 ou 2, dans lequel le liquide purifié a une concentration en solvant organique, telle que mesurée par chromatographie en phase gazeuse, inférieure ou égale à une limite de détection.

4. Procédé de production d'acide acétique purifié selon l'une quelconque des revendications 1 à 3, dans lequel la séparation est effectuée par mise en contact du liquide purifié avec la membrane de séparation (4) sous pression réduite.

5. Procédé de production d'acide acétique purifié selon l'une quelconque des revendications 1 à 4, dans lequel la membrane de séparation (4) est une ou plusieurs membranes de séparation (4) zéolites choisies dans le groupe constitué par des membranes de séparation (4) zéolites de type A, de type FAU, de type CHA, de type MFI, de type MOR et de type DDR.

6. Procédé de production d'acide acétique purifié selon l'une quelconque des revendications 1 à 5, dans lequel la solution mixte est obtenue à partir d'un liquide rejeté pendant un processus de production d'acétate de cellulose.

# FIG. 1

# FIG. 2

Acetic acid concentration X11 in the purified liquid (mass%)

FIG. 3

Acetic acid concentration X11 in the purified liquid (mass%)

Acetic acid concentration X21 in the permeated liquid (wt.%)

# FIG. 4

Acetic acid concentration X11 in the purified liquid (mass%)

# FIG. 5

Acetic acid concentration X11 in the purified liquid (mass%)

# FIG. 6

Vapor use rate of the
distillation column
(when the reference is 100)

Water concentration in the purified liquid in the
distillation column (wt.%)

# FIG. 7

FIG. 8

**EP 4 129 968 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020063081 A
- JP 9048744 A
- JP 10114699 A
- JP 2111404 A
- JP 2014226573 A
- JP H09151158 A